# EUROPEAN PATENT APPLICATION

(11) **EP 2 796 163 A1**
(43) Date of publication of application: **29.10.2014**
(21) Application number: 12860483.2
(22) Date of filing: 19.12.2012
(51) Int. Cl.: A61M 37/00

(54) **SUBCUTANEOUSLY IMPLANTED DEVICE FOR INJECTING DRUG SOLUTION**

(30) Priority: 21.12.2011 JP 2011280105
(71) Applicant: Terumo Clinical Supply Co., Ltd., Gifu 501-6024 (JP)
(72) Inventor: NAGAO, Shigeyoshi, Kakamigahara-shi Gifu 501-6024 (JP); YAMAGUCHI, Keiji, Kakamigahara-shi Gifu 501-6024 (JP); TANAKA, Seiichi, Kakamigahara-shi Gifu 501-6024 (JP)
(74) Representative: Casalonga
(86) International application number: PCT/JP2012/082949
(87) International publication number: WO 2013/094644

(57) **Abstract**

A liquid medicine injection device (1) has an upper member (3), a lower member (2), and a sealing member (4) accommodated between both members. The lower member has a part (22a), for pressing a peripheral portion of the sealing member, which is formed of an upper surface of an annular wall part (22) and an annular edge part (25) formed at an inner side of the part (22a) for pressing the peripheral portion of the sealing member. An upper-surface side of a flange part (42) of the sealing member is pressed by the upper member, and an outer-edge portion of a lower-surface side of the flange part is pressed by the part (22a) for pressing the peripheral portion of the sealing member with the annular edge part (25) in penetration into an inner-edge portion of the lower-surface side of the flange part. As a result, there is a decrease of a skirt-shaped projected part (43) of the sealing member, and an outer peripheral surface of the lower end of a part (41b) to be inserted into the lower member closely contacts an inner surface of the annular wall part.

## Description

### TECHNICAL FIELD

The present invention relates to a liquid medicine injection device for injecting a liquid medicine into a patient's body and more particularly to a liquid medicine injection device of subcutaneous implant type to be used in a medical treatment such as chemotherapy.

### BACKGROUND ART

In the treatment of cancer and the like, a liquid medicine injection therapy of injecting the liquid medicine into the body is practiced. The liquid medicine injection device of subcutaneous implant type is utilized to use for the liquid medicine injection therapy. The liquid medicine injection devices are disclosed in a patent document 1 (Japanese Published Examined Patent Application No.H04-10832, USP 4,929,236), a patent document 2 (WO 2009/35582), and a patent document 3 (Japanese Patent Application Laid-Open Publication No. 2004-350937) proposed by the present applicant.

The liquid medicine injection device has the body composed of the ring-shaped upper member and the lower member and the sealing member (septum) accommodated inside the body with the peripheral portion of the sealing member being pressed by the upper member and the lower member. The lower surface of the sealing member (septum) and the inner surface of the body form the liquid medicine injection inner space. The body has the passageway communicating with the above-described space to flow the liquid medicine out of the space. The sealing member (septum) consists of a plug made of rubber through which a liquid medicine injection needle can be inserted at a plurality of times.

### PRIOR ART DOCUMENT

### PATENT DOCUMENT

Patent document 1 Japanese Published Examined Patent Application No.H04-10832 (USP 4,929,236)
Patent document 2 WO 2009/35582
Patent document 3 Japanese Patent Application Laid-Open Publication No. 2004-350937

### SUMMARY OF THE INVENTION

### PROBLEMS TO BE SOLVED BY THE INVENTION

In the above-described conventional implant type liquid medicine injection devices disclosed in the patent documents 1 through 3, the peripheral portion of the sealing member (septum) is pressed by the upper and lower members composing the body of the liquid medicine injection device and sandwiched therebetween, with the lower portion of the septum in penetration into the lower member. The sealing member (septum) deforms because the peripheral portion thereof is pressed by the upper and lower members. Owing to the deformation, it is difficult to avoid the formation of a gap between the inner surface of the lower member and the peripheral portion of the lower surface of the sealing member (septum). It is difficult for a blood component which has penetrated into the gap to flow out of the gap, which may cause thrombus to generate.

It is an object of the present invention to provide a liquid medicine injection device of subcutaneous implant type (access port) in which a gap is not formed between the inner surface of the lower member accommodating the lower portion of the sealing member (septum) and the peripheral portion of the lower surface of the sealing member (septum) and which is capable of preventing the generation of thrombus and the like attributable to the penetration of blood into the gap.

### MEANS FOR SOLVING THE PROBLEMS

The means for achieving the object is as described below.

A liquid medicine injection device of subcutaneous implant type comprises a device body composed of a ring-shaped upper member and a lower member closing a bottom surface of said upper member, a sealing member accommodated between said upper member and said lower member, closing an opening of said upper member and forming a liquid medicine inflow space inside said body, and a discharge port communicating with said liquid medicine inflow space, wherein said lower member has a bottom plate part; an annular wall part projected from said bottom plate part; a part, for pressing a peripheral portion of said sealing member, which is formed of an outer-edge side portion of an upper surface of said annular wall part; and an annular edge part which has an annular inclined surface formed at an inner-edge side portion of said upper surface of said annular wall part, upward projecting, and upward increasing in an inner diameter thereof and which can be_inserted into said sealing member; said ring-shaped upper member has a body part accommodating said sealing member with a central portion of said sealing member being projected and an annular projected part, for pressing said peripheral portion of said sealing member, which is projected inward into said opening of said body part; said sealing member has a body part; a flange part, formed at a peripheral portion of said body part, which is to be pressed by a lower surface of said annular projected part of said upper member and by said upper surface of said annular wall part of said lower member; a part, formed below said flange part, which is to be inserted into said lower member; and a skirt-shaped projected part which is formed at a peripheral portion of a lower surface of said part to be inserted into said lower member and becomes thinner toward an end thereof; in said sealing member, an upper-surface side of said flange part thereof is pressed by said annular projected part of said upper member, and an outer-edge portion of a lower-surface side of said flange part is pressed by said part, for pressing said peripheral portion of said sealing member, which is formed of said upper surface of said annular wall part of said lower member, said annular edge part formed on said annular wall part of said lower member is penetrating into an inner-edge portion of said lower-surface side of said flange part; said sealing member deforms owing to a high compression applied thereto by said upper member and said lower member sandwiching said sealing member therebetween and owing to said penetration of said annular edge part of said lower member into said inner-edge portion of said lower-surface side of said flange part, so that there is a decrease in a height of said flange part, an increase in a height of said part to be inserted into said lower member, and a decrease of said skirt-shaped projected part, and an outer peripheral surface of a lower end of said part to be inserted into said lower member closely contacts an inner surface of said annular wall part of said lower member.

### BRIEF DESCRIPTION OF THE DRAWINGS

Fig. 1 is a perspective view of a liquid medicine injection device of the present invention.
Fig. 2 is a plan view of the liquid medicine injection device of the present invention
Fig. 3 is a front view of the liquid medicine injection device shown in Fig. 2.
Fig. 4 is a sectional view taken along a line A-A of Fig. 2.
Fig. 5 is a sectional view taken along a line B-B of Fig. 3.
Fig. 6 is a front view of a sealing member (septum) for use in the liquid medicine injection device of the present invention.
Fig. 7 is a bottom view of the sealing member shown in Fig. 6.
Fig. 8 is a sectional view taken along a line C-C of Fig. 6.
Fig. 9 is a plan view of a lower member for use in the liquid medicine injection device of the present invention.
Fig. 10 is a sectional view taken along a line D-D of Fig. 9.
Fig. 11 is an explanatory view for explaining a state before the liquid medicine injection device of the present invention is fixed.
Fig. 12 is an external view of the liquid medicine injection device of the present invention on which a catheter has been mounted.
Fig. 13 is a plan view of a liquid medicine injection device of another embodiment of the present invention.
Fig. 14 is a sectional view taken along a line E-E of Fig. 13.
Fig.15 is a bottom view of the liquid medicine injection device shown in Fig. 13.

### MODE FOR CARRYING OUT THE INVENTION

The liquid medicine injection device of subcutaneous implant type of the present invention is described below by using embodiments shown below in the drawings.

A liquid medicine injection device of subcutaneous implant type 1 of the present invention has a device body composed of a ring-shaped upper member 3 and a lower member 2 closing a bottom surface of the upper member 3, a sealing member 4 accommodated between the upper member 3 and the lower member 2, closing an opening of the upper member 3, and forming a liquid medicine inflow space 10 inside the device body, and a discharge port 5 communicating with the liquid medicine inflow space 10.

The lower member 2 has a bottom plate part 21; an annular wall part 22 projected from the bottom plate part 21; a part 22a for pressing a peripheral portion of the sealing member formed of an outer-edge side portion of an upper surface of the annular wall part 22; and an annular edge part 25 which has an annular inclined surface formed at an inner-edge side portion of the upper surface of the annular wall part 22, upward projecting, and upward increasing in an inner diameter thereof and which can be inserted into the sealing member 4.

The ring-shaped upper member 3 has a body part 31 accommodating the sealing member 4 with a central portion of the sealing member 4 being exposed (projected) and an annular projected part 32, for pressing the peripheral portion of the sealing member 4, which is projected inward into the opening of the body part 31.

The sealing member 4 has a body part 41; a flange part 42, formed at a peripheral portion of the body part 41, which is to be pressed by a lower surface of the annular projected part 32 of the upper member 3 and by the upper surface of the annular wall part 22 of the lower member; a part 41b, formed below the flange part 42, which is to be inserted into the lower member, and a skirt-shaped projected part 43 which is formed at a peripheral portion of a lower surface of the part 41b to be inserted into the lower member and becomes thinner toward an end thereof.

In the sealing member, an upper-surface side of the flange part 42 thereof is pressed by the annular projected part 32 of the upper member 3, and an outer-edge portion of a lower-surface side of the flange part 42 is pressed by the part 22a, for pressing the peripheral portion of the sealing member, which is formed of the upper surface of the annular wall part 22 of the lower member 2 with the annular edge part 25 formed on the annular wall part 22 of the lower member 2 in penetration into an inner-edge portion of the lower-surface side of the flange part 42. The sealing member 4 deforms owing to a high compression applied thereto by the upper member 3 and the lower member 2 sandwiching the sealing member 4 therebetween and owing to the penetration of the annular edge part 25 of the lower member 2 into the inner-edge portion of the lower-surface side of the flange part 42. As a result, there is a decrease in the height of the flange part 42, an increase in the height of the part 41b to be inserted into the lower member, a decrease of the skirt-shaped projected part 43 of the sealing member 4, and an outer peripheral surface of the lower end of the part 41b to be inserted into the lower member closely contacts an inner surface of the annular wall part 22 of the lower member 2.

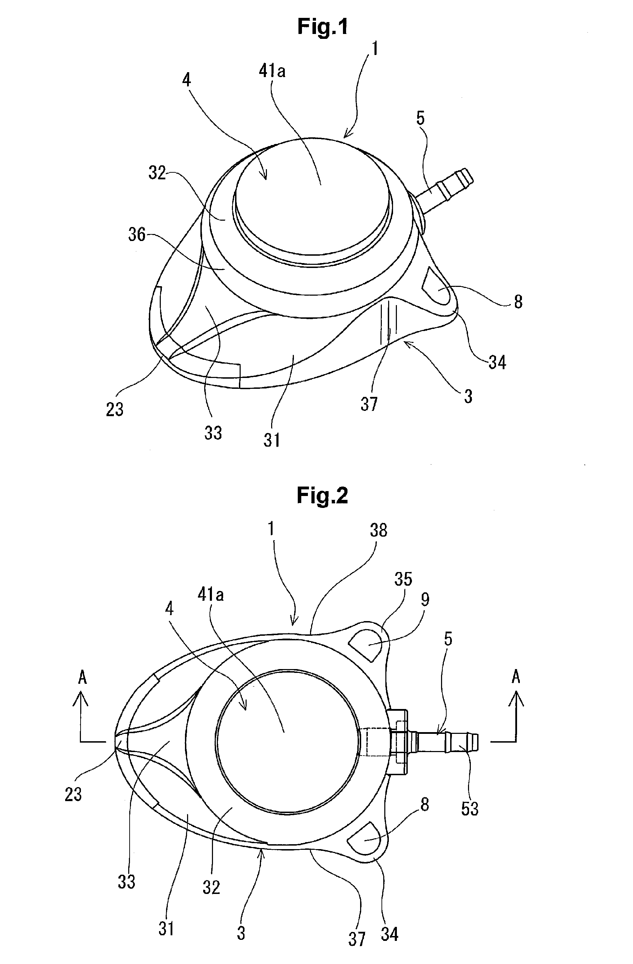

As shown in Figs. 1 through 5, the liquid medicine injection device of subcutaneous implant type 1 of this embodiment is constructed of the device body and the sealing member 4. The device body has the lower member 2 having a concave portion forming the liquid medicine inflow space 10; the ring-shaped upper member 3 mounted on an upper-surface side of the lower member 2; and the discharge port 5 mounted on a side of the upper member 3. The sealing member 4 is disposed between the lower member 2 and the upper member 3. The sealing member 4 is interposed between the lower member 2 and the upper member 3. The flange part 42 of the sealing member 4 is pressed by the lower member 2 and the upper member 3 and liquid-tightly seals the space between the lower member 2 and the upper member 3.

The ring-shaped upper member 3 has the ring-shaped body part 31 accommodating the sealing member 4 therein with the central portion of the sealing member 4 being exposed and the annular projected part 32 projected inward into the opening of the body part 31 (projected toward the center of the opening) from an upper portion of the body part 31. The annular projected part 32 is formed to press the upper surface of the peripheral portion (flange part) 42 of the sealing member 4 by its lower surface. In the liquid medicine injection device of this embodiment, the annular projected part 32 has an annular edge portion 39 having an annular inclined surface which is formed on an inner-edge side portion of the lower surface of the annular projected part 32, projecting downward, and downward increasing in its inner diameter. The annular edge portion 39 can be inserted into the upper surface of the flange part of the sealing member 4. The annular projected part 32 has an annular portion 32a, for pressing the peripheral portion of the sealing member, which is formed at an outer side of the annular edge portion 39 as a flat surface. The portion 32a for pressing the peripheral portion of the sealing member presses (compresses) the flange part 42 of the sealing member 4.

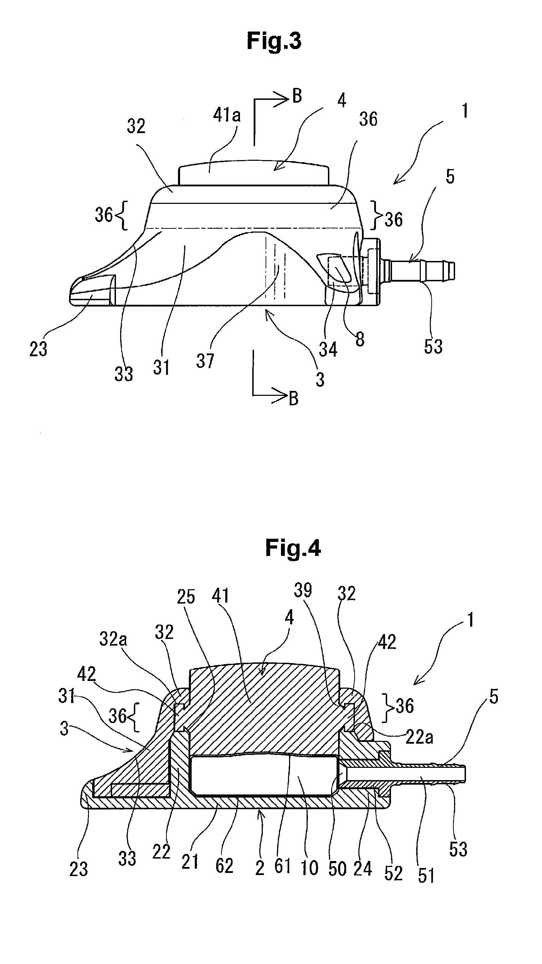

As shown in Figs. 2, 3, and 5, the upper member 3 has side bulged parts 34 and 35 formed at both sides of a front end portion thereof. The discharge port 5 is interposed between the side bulged parts 34 and 35. As shown in Figs. 1 and 3 (particularly Fig. 1), the upper member 3 has opposed gripping erect side surface parts 37 and 38 formed between a rear end portion 33 thereof and the side bulged part 34 as well as the side bulged part 35. The gripping erect side surface parts 37 and 38 are formed as an almost erect surface respectively and are curved much more greatly than other portions of the upper member 3 in the neighborhood of the side bulged parts 34 and 35. The center of the sealing member 4 is positioned between the opposed gripping erect side surface parts 37 and 38. In the liquid medicine injection device of this embodiment, the central portion of the sealing member 4 is positioned between greatly curved portions, of the gripping erect side surface parts 37 and 38, which are disposed in the neighborhood of the side bulged parts 34 and 35.

The upper member 3 has the rear end portion 33 at a position opposed to the discharge port 5. In other words, the rear end portion 33 is extended to the side opposite to the discharge port 5 from the outer edge of the annular projected part. The rear end portion 33 becomes thinner toward its rear end and narrower. As shown in Fig. 2, in the liquid medicine injection device of this embodiment, the rear end portion 33 is a crescent shape that is slightly crushed.

As shown in Figs. 1 and 3, the erect side surface parts 37 and 38 are extended from the rear end portion 33 toward the side bulged parts 34 and 35 respectively, become gradually larger in the height thereof, become highest in the neighborhood of the side of a central portion of the annular projected part 32, and become suddenly lower toward the side bulged parts 34 and 35 respectively. Because the gripping erect side surface parts 37 and 38 are highest in the neighborhood of the side of the central portion of the annular projected part 32, an operator can easily grip the side of the central portion of the annular projected part 32. Further when the operator grips the side of the central portion of the annular projected part, operator's fingers feel the portions, of the gripping erect side surface parts 37 and 38, which are greatly curved in the neighborhood of the side bulged parts 34 and 35. Thus the operator can easily recognize that the central portion of the annular projected part 32 (in other words, the central portion of the sealing member 4) is disposed between gripped portions. The formation of the gripping erect side surface parts 37 and 38 for the upper member 3 allows a liquid medicine injection needle insertion operation to be easily performed after the liquid medicine injection device is implanted subcutaneously and the liquid medicine injection device to be handled easily in a surgical procedure of embedding the liquid medicine injection device subcutaneously.

The upper member 3 has a cylindrical part 36 formed between the erect side surface parts 37 and 38 and the upper end of the upper member 3. The cylindrical part 36 is almost cylindrical or gently decreases a little toward its upper end in its diameter. As shown in Fig. 3, in the liquid medicine injection device of this embodiment, the cylindrical part 36 gently decreases toward its upper end. The formation of the cylindrical part 36 for the upper member 3 may allow the operator to recognize the cylindrical part 36 (in other words, the central portion of the annular projected part 32, the central portion of the sealing member 4) through the skin after the liquid medicine injection device is implanted subcutaneously. The formation of the cylindrical part 36 for the upper member 3 also allows the operator to easily grip the upper portion of the liquid medicine injection device 1. Gripping the upper portion of the liquid medicine injection device 1 allows the operator to easily recognize the sealing portion and securely grip the liquid medicine injection device 1. Therefore the operator can quickly perform the liquid medicine injection needle insertion operation.

As shown in Figs. 1 through 3 and 5, each of the side bulged parts 34 and 35 becomes gradually thinner toward the end thereof and has a through-hole penetrating therethrough from the upper surface thereof to the lower surface thereof. Elastic members 8 and 9 through which a surgical needle can be inserted are implanted in the through-holes. The side bulged parts 34 and 35 serve as portions to be sewed to a living body.

The elastic members 8, 9 are formed of an elastic material respectively. Examples of materials for the elastic members include rubbers such as silicone rubber, isoprene rubber, and natural rubber; and resins such as polyurethane, polyamide elastomer, polybutadiene, and soft vinyl chloride or combinations of not less than two of these materials. Of these materials, the silicone rubber is preferable because it is inert for living bodies and has a comparatively small change in its physical property.

As shown in Fig. 2, as the form of the bottom of the upper member of this embodiment, both sides thereof at its proximal side are bulged. Thus the bottom form of the upper member is semi-elliptic. The center of the upper surface of the upper member is positioned near the proximal side of the bottom form thereof and is circular.

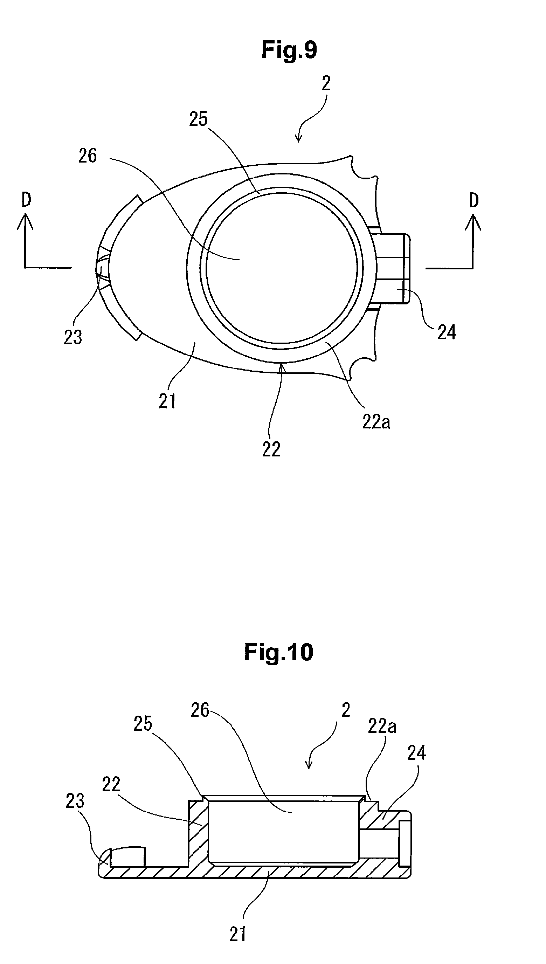

As shown in Figs. 3, 5, 9, and 10, the lower member 2 has the bottom plate part 21; the annular wall part 22 projected from the bottom plate part 21; and a rear end portion 23. The annular wall part 22 is almost cylindrical and has a concave portion 26 for forming the liquid medicine inflow space 10 therein. In addition the annular wall part 22 has a discharge port mounting portion 24, communicating with the concave portion 26, which is disposed at a lower portion of its side surface. The discharge port mounting portion 24 is a cylindrical portion projected from the annular wall part 22 of the lower member 2 and has a lumen into which a proximal portion of the discharge port 5 can be inserted. As shown in Fig. 9, the bottom plate part 21 is missing at both sides of its proximal portion and thus has a semi-elliptic configuration. The center of the annular wall part is disposed near the proximal side of the lower member 2 in the form of the bottom surface of the lower member 2.

In the liquid medicine injection device of this embodiment, the lower member 2 has the part 22a, for pressing the peripheral portion of the sealing member, which is formed of the outer-edge side portion of the upper surface of the annular wall part 22. In addition, the lower member 2 has the annular edge part 25 having the annular inclined surface which is formed at the inner-edge side portion (in other words, the inner side of the part 22a for pressing the peripheral portion of the sealing member) of the upper surface of the annular wall part 22. The annular inclined surface is projected upward and increases upward in its inner diameter. The annular edge part 25 can be inserted into the lower surface of the flange part 42 of the sealing member 4.

The sealing member 4 has the columnar body part 41 and the annular flange part 42 formed at the peripheral portion of the body part by projecting the annular flange part 42 from the body part. The flange part 42 is the portion to be pressed by the lower surface of the annular projected part 32 of the upper member 3 and by the upper surface of the annular wall part 22 of the lower member. The sealing member 4 of this embodiment has the part 41b, to be inserted into the lower member, which is formed below the flange part 42 and has a small outer diameter than that of the flange part and an inner diameter almost equal to that of the annular wall part and a part 41a, to be inserted into the upper member, which is formed above the flange part 42 and has a smaller outer diameter than that of the flange part and an inner diameter almost equal to or a little smaller than that of the annular wall part of the upper member 3.

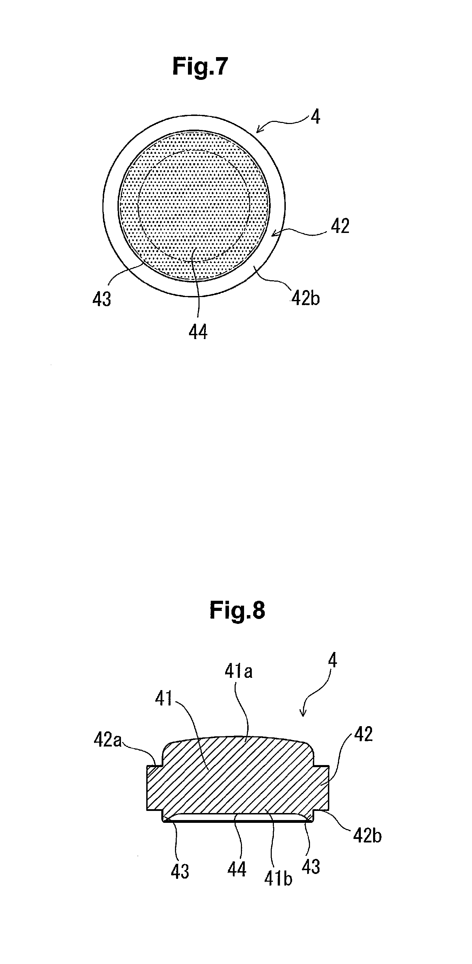

The sealing member 4 has the skirt-shaped projected part 43 which is formed at the peripheral portion of the lower surface 44 of the part 41b to be inserted into the lower member and becomes thinner toward the end thereof. As shown in Fig. 8, the skirt-shaped projected part 43 becomes thinner toward its lower end and increases in its inner diameter. Therefore the inner surface of the skirt-shaped projected part 43 is formed as an inclined surface which increases in its inner diameter toward its lower end (specifically, the curved inclined surface which increases in its inner diameter toward its lower end).

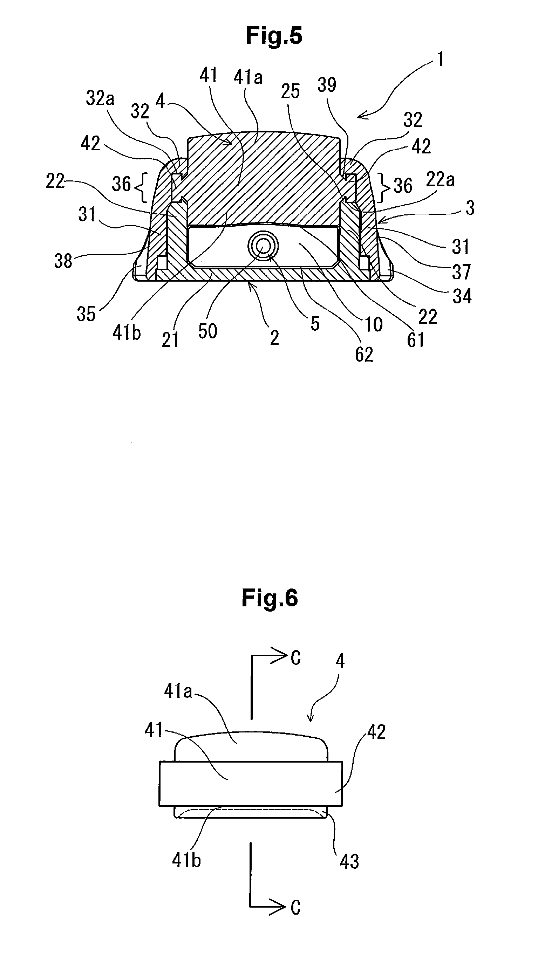

The part 41a of the sealing member 4 to be inserted into the upper member 3 is inserted into the opening thereof. As shown in Figs. 1 through 5, the upper end portion of the part 41a to be inserted into the upper member is projected above the upper surface of the upper member 3. As shown in Figs. 4 and 5, a portion of the part 41b of the sealing member 4 to be inserted into the lower member is penetrated into the annular wall part 22 of the lower member 2 and accommodated therein. The liquid medicine inflow space 10 is formed between the lower surface 44 of the part 41b of the sealing member 4 to be inserted into the lower member and the inner surface of the concave portion 26 of the lower member 2.

As materials for the device body of the liquid medicine injection device (composed of the lower member 2 and the upper member 3), those resistant to chemicals and biocompatible are preferable. Polysulfone, polyether sulfone, epoxy resin, and polyacetal are used.

The liquid medicine injection needle can be inserted through the sealing member 4. An inserted portion of the sealing member is sealed after the liquid medicine injection needle is removed therefrom. The sealing member 4 is formed of an elastic material. Examples of materials for forming the sealing member 4 include rubbers such as silicone rubber, isoprene rubber, and natural rubber; and resins such as polyurethane, polyamide elastomer, polybutadiene, and soft vinyl chloride or combinations of not less than two of these materials. Of these materials, the silicone rubber is preferable because it is inert for living bodies and has a comparatively small change in its physical property.

As shown in Figs. 6 through 8, in the liquid medicine injection device of this embodiment, an upper-surface side 42a of the flange part 42 is pressed by the annular projected part 32 of the upper member 3, and the outer-edge portion of a lower-surface side 42b of the flange part 42 is pressed by the part 22a, for pressing the peripheral portion of the sealing member, which is formed on the upper surface of the annular wall part 22 of the lower member 2 with the annular edge part 25 of the annular wall part 22 of the lower member 2 in penetration into the inner-edge portion of the lower-surface side of the flange part 42.

In the liquid medicine injection device of this embodiment, the outer-edge portion of the upper-surface side of the flange part 42 is pressed by the portion 32a, for pressing the peripheral portion of the sealing member, which is disposed on the lower surface of the annular projected part 32 of the upper member 3 with the annular edge portion 39 of the annular projected part 32 of the upper member 3 in penetration into the inner-edge portion of the upper-surface side of the flange part 42.

As described above, because in the liquid medicine injection device 1 of this embodiment, the annular edge parts 25 and 39 are in penetration into the lower surface and upper surface of the flange part 42 of the sealing member 4 respectively, the sealing member can be securely gripped and securely holds a liquid-tight state.

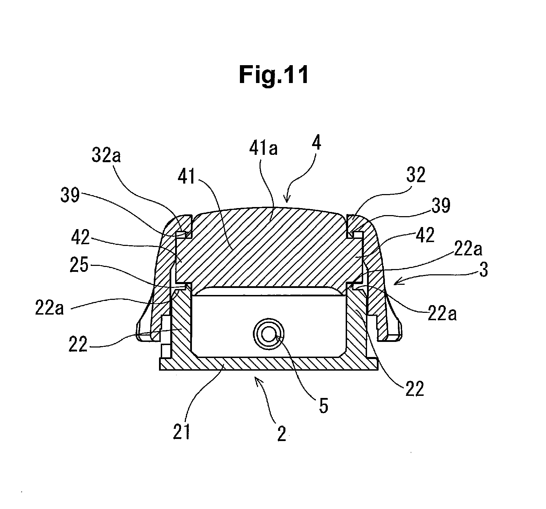

Owing to a high compression (more specifically, high compression owing to the pressurization applied to the flange part by the part 22a, of the lower member, for pressing the peripheral portion of the sealing member and the pressurization applied to the flange part by the portion 32a, of the upper member 3, for pressing the peripheral portion of the sealing member) applied thereto by the upper member 3 and the lower member 2 and owing to the penetration of the annular edge part 25 of the lower member 2 into the flange part 42, the sealing member 4 deforms from a state shown in Figs. 6 through 8 and 11 to a state shown in Figs. 4 and 5. Fig. 11 is an explanatory view for explaining a state before the upper and lower members of the liquid medicine injection device 1 are fixed to each other. As shown in Fig. 11, in a state in which the sealing member 4 is placed on the lower member 2 and the sealing member 4 is covered with the upper member, the lower surface of the upper member 3 and the upper surface of the lower member 2 are spaced at a predetermined distance, with the upper member 3 out of contact with the lower member 2. Because the sealing member 4 is compressed by the upper member 3 and the lower member 2 sandwiching the sealing member 4 therebetween, the sealing member deforms from the state shown in Fig. 11 to the state shown in Figs. 4 and 5, so that the upper member 3 and the lower member 2 are fixed to each other with both members in contact with each other.

Owing to the deformation of the sealing member 4 from the state shown in Fig. 11 to the state shown in Figs. 4 and 5, there is a decrease in the height of the flange part 42 of the sealing member 4 by 20 to 45%, preferably 25 to 40%, an increase in the height of the part 41b thereof to be inserted into the lower member by 1.5 to 3 times, an increase in the height of the part 41a thereof to be inserted into the upper member by 1.3 to 2 times, and an increase in the height of the sealing member at the central portion (thickness) thereof by 1.5 to 2.5 times.

Owing to the decrease in the height of the flange part 42 and the increase in the height of the part 41b to be inserted into the lower member, there is an increase in the penetration depth of the part 41b to be inserted into the lower member. Similarly owing to the decrease in the height of the flange part 42 and the increase in the height of the part 41a to be inserted into the upper member, there is an increase in the penetration depth of the part 41a to be inserted into the opening of the upper member. As shown in Figs. 4 and 5, the upper portion of the part 41a to be inserted into the upper member is projected above the opening of the upper member 3. Further as shown in Figs. 4 and 5, there is a decrease of the skirt-shaped projected part 43 of the sealing member 4, and the outer peripheral surface of the lower end of the part 41b to be inserted into the lower member closely contacts the inner surface of the annular wall part 22 of the lower member 2.

Further in the liquid medicine injection device of this embodiment, owing to the deformation of the sealing member 4 caused by a high compression (more specifically, compression which decreases the height (thickness) of the flange part by not less than 20%) applied thereto by the upper member 3 and the lower member 2 and caused by the penetration of the annular edge part 25 of the lower member 2 into the flange part 42, the skirt-shaped projected part 43 of the sealing member 4 almost disappears. Owing to the high compression applied to the upper and lower surfaces of the flange part 42 of the sealing member 4, it is supposed that the height (thickness) of the flange part of the sealing member decreases and that the height (thickness) of the entire (central portion) sealing member increases.

Owing to the increase in the thickness of the sealing member caused by the above-described high compression, the side surface of the part 41b of the sealing member 4 to be inserted into the lower member is pressed against the inner surface of the annular wall part 22 of the lower member 2. Thereby it is considered that the close contact between both members can be ensured.

When the annular edge part 25 of the lower member 2 is inserted into an inner-side corner of the lower surface of the flange part 42 of the sealing member 4 without forming the skirt-shaped projected part 43 for the sealing member, the neighborhood of the inner-side corner is pulled outward. Owing to the pulling, the side surface of the part 41b of the sealing member 4 to be inserted into the lower member is pulled upward. Thereby there is a high possibility that a gap is formed between the lower end of the side surface of the part 41b to be inserted into the lower member and the inner surface of the annular wall part 22 of the lower member 2.

In the liquid medicine injection device 1 of this embodiment, owing to the above-described deformation of the sealing member 4, the lower surface of the part 41b of the sealing member 4 to be inserted into the lower member is a little recessed at its central portion. Because the lower surface of the part 41b of the sealing member 4 to be inserted into the lower member is recessed at its central portion, the central portion of the liquid medicine inflow space 10 is higher than other portions thereof.

In the liquid medicine injection device 1 of this embodiment, the liquid medicine inflow space 10 is formed as a columnar space, having a predetermined height, which is formed of the inner surface of the sealing member 4 and the inner surface of the concave portion 26 (the inner surface of the annular wall part 22 and the upper surface of the bottom plate part 21).

In the sealing member 4 of this embodiment, the lower surface of the part 41b to be inserted into the lower member is formed as a rough surface, more specifically, an uneven surface or a stain finish surface. The rough surface can be formed by performing embossing by using blasting of a portion, of a sealing member forming die, for forming the lower surface of the part of the sealing member to be inserted into the lower member or forming the sealing member by laser processing.

In this embodiment, the lower surface 44 of the part 41b of the sealing member 4 to be inserted into the lower member is coated with an antithrombotic compound 61. The lower surface 44 of the part 41b to be inserted into the lower member may be coated with the antithrombotic compound 61 without forming the uneven surface or the stain finish surface thereon. But it is preferable to coat the lower surface 44 of the part 41b to be inserted into the lower member with the antithrombotic compound 61 after forming the uneven surface or the stain finish surface thereon. By so doing, it is possible to securely coat the lower surface 44 of the part 41b to be inserted into the lower member with the antithrombotic compound, and further the antithrombotic compound little peels therefrom. It is preferable to coat the inner surface of the lower member 2 forming the liquid medicine inflow space 10 with an antithrombotic compound 62.

As the antithrombotic compound, it is possible to use heparin, polyalkylsulfone, ethyl cellulose, acrylic acid ester copolymer, methacrylic acid ester copolymer (for example, polyHEMA[polyhydroxyethyl methacrylate]), a block or a graft copolymer having a hydrophobic segment and a hydrophilic segment (for example, a block copolymer of HEMA-styrene-HEMA, a block copolymer of HEMA-MMA[methyl methacrylate], a block copolymer of HEMA-LMA[lauryl methacrylate], a block copolymer of PVP[polyvinylpyrrolidone]-MMA, a block copolymer of HEMA-MMA/AA[acrylic acid]), a blended polymer consisting of a mixture of any one of the above-described block copolymers and a polymer having an amino group, fluorine-containing resin, and a synthetic polymer containing alkoxyalkyl (meta)acrylate consisting of an alkoxy group having a carbon number of 1 to 4 and an alkyl group having a carbon number of 1 to 4 as its main component. The synthetic polymer containing the alkoxyalkyl (meta)acrylate as its main component, the block copolymer of HEMA-styrene-HEMA, the block copolymer of HEMA-MMA[methyl methacrylate], and the block copolymer of HEMA-MMA/AA[acrylic acid] are preferable.

The synthetic polymer containing the alkoxyalkyl (metha)acrylate as its main component is a homopolymer or a copolymer consisting of one kind or not less than two kinds of alkoxyalkyl (metha)acrylates shown below or a copolymer of the alkoxyalkyl (metha)acrylate and monomers copolymerizable therewith.

The alkoxyalkyl (metha)acrylate contains both of alkoxyalkyl acrylate and alkoxyalkyl methacrylate. Examples of the alkoxyalkyl (metha)acrylate include methoxy methyl acrylate, methoxy ethyl acrylate, methoxy propyl acrylate, ethoxy methyl acrylate, ethoxy ethyl acrylate, ethoxy propyl acrylate, ethoxy butyl acrylate, propoxy methyl acrylate, butoxy ethyl acrylate, methoxy butyl acrylate, methoxy methyl methacrylate, methoxy ethyl methacrylate, ethoxy methyl methacrylate, ethoxy ethyl methacrylate, propoxy methyl methacrylate, and butoxy ethyl methacrylate. Of these alkoxyalkyl (metha)acrylates, the methoxy ethyl acrylate is preferable.

Examples of monomers copolymerizable with the alkoxyalkyl (metha)acrylate include methyl acrylate, ethyl acrylate, propyl acrylate, butyl acrylate, 2-ethylhexyl acrylate, methyl methacrylate, ethyl methacrylate, butyl methacrylate, hexyl acrylate, hexyl methacrylate, ethylene, and propylene.

Copolymerizable monomers not having a hydroxyl group or a cationic group in the molecule thereof are preferable. As the copolymer, any of a random copolymer, a block copolymer, and a graft copolymer will do. The copolymer can be synthesized by known methods such as radical polymerization, ion polymerization, and macromer polymerization.

It is preferable that in any of the copolymers, the ratio of the monomer copolymerizing with the alkoxyalkyl (metha)acrylate is not more than 50%. When the ratio of the monomer exceeds 50%, the effect of the alkoxyalkyl (metha)acrylate is liable to deteriorate. The weight-average molecular weight of the alkoxyalkyl (metha)acrylate obtained in this manner is favorably 10,000 to 1,000,000 and preferably 20,000 to 100,000.

After coating a blood contact surface with a hydrophilic resin except the above-described heparin, the heparin may be fixed to the surface of the hydrophilic resin. In this case, to fix the heparin to the surface of the hydrophilic resin, it is preferable that the hydrophilic resin has any one of a hydroxyl group, an amino group, a carboxyl group, an epoxy group, an isocyanate group, an epoxy group, a thiocyanate group, an acid chloride group, an aldehyde group, and a carbon-carbon double bond or has a group which can be easily convertible to these groups. It is especially preferable to use a blended polymer consisting of the mixture of the hydrophilic resin and a polymer having the amino group. As the polymer having the amino group, polyamine and particularly PEI [polyethyenimine] are preferable.

To fix the heparin to a portion, after the portion to which the hydrophilic resin is to be fixed is coated with the hydrophilic resin, a heparin aqueous solution is brought into contact with the surface of the hydrophilic resin. Thereafter the hydrophilic resin is brought into contact with a fixing agent such as aldehydes including glutaraldehyde, terephthalaldehyde, formaldehyde, diphenylmethane diisocyanate, 2,4-tolylene diisocyanate, carbodiimide-modified diphenylmethane diisocyanate, epichlorohydrin, 1,4-butanediol diglycidyl ether or poly(ethylene glycol) diglycidyl ether to allow the hydrophilic resin to bind covalently. Thereby the heparin can be fixed to the portion.

As shown in Figs. 1 through 5, the discharge port 5 is mounted on the liquid medicine injection device 1 of this embodiment. The discharge port 5 is cylindrical and has a catheter proximal portion mounting part 53 disposed at its distal side, a lower member mounting part 52 disposed at its proximal side, and a flow passageway 51 disposed inside the discharge port 5. A proximal-end opening 50 is disposed at the proximal end of the flow passageway 51 and at the proximal end of the lower member mounting part 24.

An annular projected portion for preventing the removal of a catheter is formed on an outer surface of the catheter proximal portion mounting part 53. The lower member mounting part 52 is inserted into the discharge port mounting portion 24 of the lower member 2 and liquid-tightly fixed thereto.

As materials for the discharge port 5, titanium, a titanium alloy, and stainless steel can be used. The titanium and the titanium alloy are preferable.

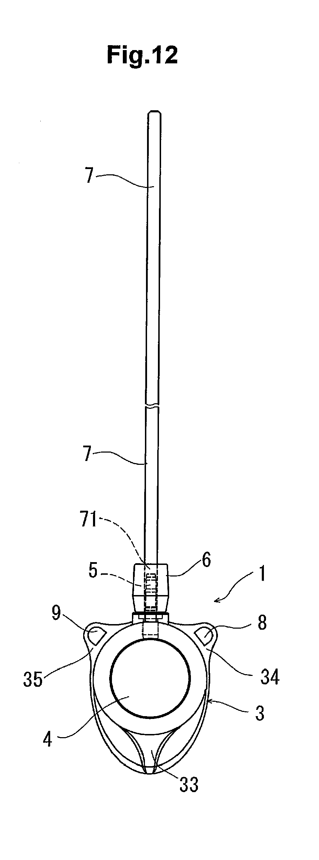

When the liquid medicine injection device 1 of the present invention is used, a catheter 7 and a protector 6 as shown in Fig. 12 are connected thereto. A proximal portion 71 of the catheter 7 is mounted on the discharge port 5 in such a way as to enclose the catheter proximal portion mounting part 53 therein. The protector 6 is fitted on the proximal portion 71 of the catheter 7 mounted on the catheter proximal portion mounting part 53 and presses the proximal portion 71 to prevent the catheter from being removed from the discharge port 5. The catheter 7 whose front end portion can be inserted into a body (specifically, blood vessels (veins or arteries), vascular channels such as bile duct, urinary duct, epidurally, subarachnoid, and abdominal cavity) is used.

The catheter 7 consists of a tubular body having an opening disposed at its front-end side and an internal lumen and has an equal outer diameter and an equal inner diameter over the entire length thereof. The outer diameter of the catheter is favorably 0.3 to 5mm and more favorably 0.9 to 2.8mm. The inner diameter of the catheter is favorably 0.1 to 2.6mm and more favorably 0.6 to 1.8mm

The catheter 7 flexible and elastic to some extent is preferably used. The catheter is formed of flexible polymeric materials including olefin-based elastomer (for example, polyethylene elastomer, polypropylene elastomer), polyester such as polyethylene terephthalate, soft polyvinyl chloride, polyurethane, urethane elastomer, polyamide, amide elastomer (for example, polyamide elastomer), fluororesin elastomer, ethylene-vinyl acetate copolymer, and silicone rubber.

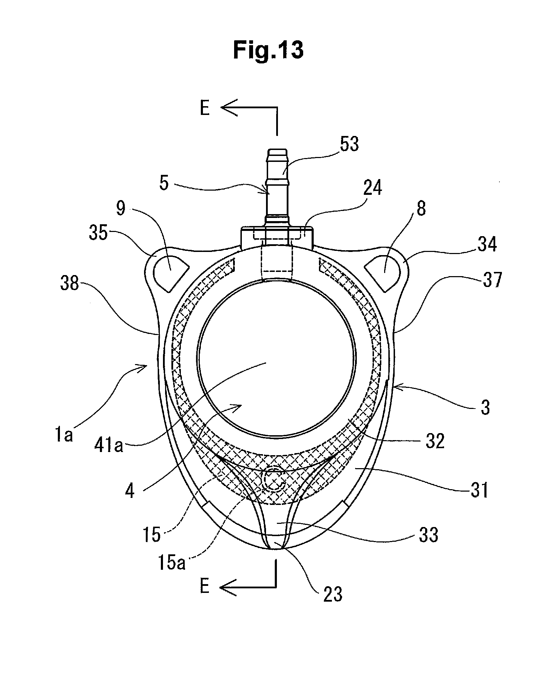

Similar to the liquid medicine injection device of subcutaneous implant type 1 as described above, the liquid medicine injection device of the present invention may have an x-ray contrast function like a liquid medicine injection device 1a shown in Figs. 13,14, and 15.

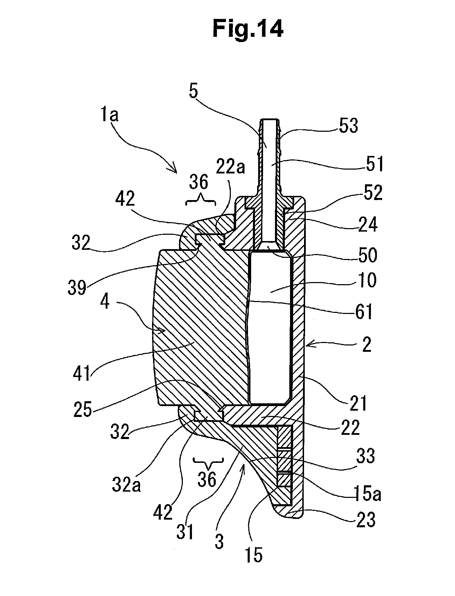

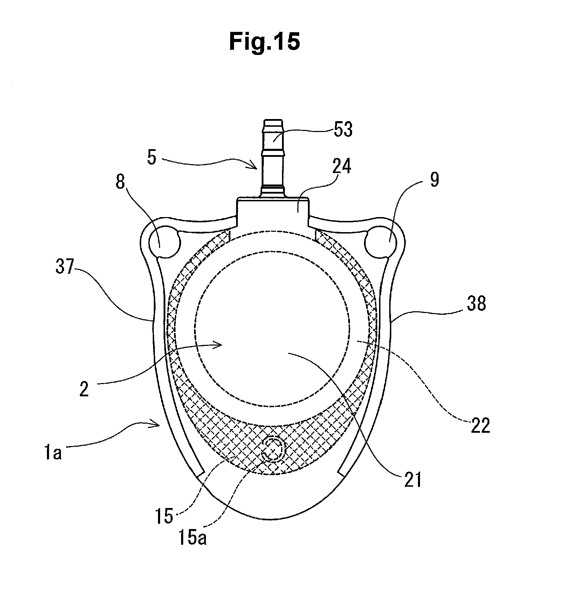

Fig. 13 is a plan view of the liquid medicine injection device 1a of another embodiment of the present invention. Fig. 14 is a sectional view taken along a line E-E of Fig. 13. Fig. 15 is a bottom view of the liquid medicine injection device shown in Fig. 13.

The liquid medicine injection device of subcutaneous implant type 1a of this embodiment has the device body composed of the ring-shaped upper member 3 and the lower member 2 closing the bottom surface of the upper member 3, the sealing member 4 accommodated between the upper member 3 and the lower member 2, closing the opening of the upper member 3, and forming the liquid medicine inflow space 10 inside the device body, and the discharge port 5 communicating with the liquid medicine inflow space 10. In addition, the liquid medicine injection device 1a has a ring-shaped contrast member 15 accommodated inside its body, more specifically, at the gap formed between the ring-shaped upper member 3 and the lower member 2. The contrast member 15 allows the liquid medicine injection device 1a to have an x-ray contrast function. The ring-shaped upper member 3, the lower member 2, the sealing member 4, and the discharge port 5 are the same as those described above.

In the liquid medicine injection device 1a of this embodiment, the contrast member 15 is formed as a thin ring-shaped member having a missing portion at the side of the discharge port 5. More specifically, as shown in Figs. 13 through 15, the contrast member 15 has an almost elliptic outer edge and an almost perfectly circular inner edge and the missing portion at the side of the discharge port 5. The contrast member 15 surrounds the outer periphery of the lower portion of the annular projected wall part 22 of the lower member 2. The sealing member 4 is positioned above the central portion of the contrast member 15. Thereby the presence of the sealing member 4 (insertable portion) can be determined by radiographic visualization.

Similarly to the above-described liquid medicine injection device 1, in the liquid medicine injection device 1a of this embodiment, the flange part 42 of the sealing member 4 is pressed by the lower surface of the annular projected part 32 of the upper member 3 and the upper surface of the annular wall part 22 of the lower member 2. As a result, the height of the flange part 42 decreases by 20 to 45% (preferably 20 to 40%). That is, the flange part 42 is sandwiched between the upper member 3 and the lower member 2 and pressed (high compression) thereby. Therefore the liquid medicine injection device is of a so-called high pressure-resistant type which does not allow liquid leak to occur, even though a liquid medicine is injected thereinto at a high pressure up to a certain extent. That is, the liquid medicine injection device of this embodiment can be used as an automatic injection port of a contrast medium in an x-ray contrast CT in which the contrast medium is administered to a patient. In the liquid medicine injection device 1a of this embodiment, the contrast member 15 has a clipped portion 15a consisting of a mark which allows association of the x-ray contrast CT in which the contrast medium is administered to the patient. More specifically, the clipped portion 15a consists of a character "C". The clipped portion 15a may consist of a character "CT". Because the contrast member 15 has the clipped portion 15a consisting of the mark which allows the x-ray contrast CT to be associated, it is possible to recognize the mark which allows the x-ray contrast CT to be associated in the radiographic visualization and recognize that the liquid medicine injection device implanted subcutaneously can be used as the automatic injection port of the contrast medium in the x-ray contrast CT.

The liquid medicine injection device 1a of the present invention has the rear end portion 33 which becomes thinner toward the rear end thereof. In correspondence to this configuration, the contrast member 15 is wide at the central portion of its rear side. Therefore by performing the radiographic visualization, it is possible to check the position of the rear end portion of the liquid medicine injection device 1a, namely, the placed state of the liquid medicine injection device 1a. The width of the contrast member 15 of this embodiment becomes gradually larger from both sides thereof toward the rear central portion thereof and is largest at the center thereof. The rear central portion of the contrast member 15 having a large width is opposite to the missing portion (discharge port 5) thereof.

The contrast member 15 of the liquid medicine injection device 1a of this embodiment is formed as the thin ring-shaped member having the missing portion at the side of the discharge port 5. Although the contrast member 15 is accommodated at the gap between the ring-shaped upper member 3 and the lower member 2, the portion where the contrast member is accommodated is not limited to this form. For example, the contrast member 15 may be embedded in the upper surface of the lower member 2, the lower surface of the lower member or the bottom surface of the upper member.

In the liquid medicine injection device 1a of this embodiment, the contrast member 15 has a reverse recognition function. More specifically, the contrast member 15 has the above-described clipped portion 15a at its above-described wide portion. The clipped portion 15a is left-right asymmetric. As described above, the clipped portion 15a consists of the character "C". In a normal placing mode in which the sealing member 4 (insertable portion) is positioned at an upper side of the liquid medicine injection device, the character "C" can be recognized in the radiographic visualization, as shown in Fig. 13. When the contrast member 15 is reversed and thus the sealing member 4 (insertable portion) is positioned at a lower side of the liquid medicine injection device, the left-right asymmetric mark ("C" in this embodiment) is reversed, as shown in the rear view of Fig. 15. Thus the character "C" cannot be recognized. Thereby the formation of the reverse recognition function 15a for the contrast member 15 allows the reverse (turning inside out) of the liquid medicine injection device 1a to be recognized in the radiographic visualization.

As materials for the contrast member 15, it is possible to use metal plates plated with metals such as gold, platinum, tungsten, titanium, titanium alloy, stainless steel, gold, platinum having a high x-ray contrast function and resin plates to which an x-ray contrast substance (an x-ray contrast substance such as powder of metal including gold, platinum, tungsten, and barium sulfate, bismuth subcarbonate, and the like) has been added. Metal plates consisting of gold, tungsten, titanium or titanium alloy and metal plates plated with gold or platinum are especially preferable.

### INDUSTRIAL APPLICABILITY

The liquid medicine injection device of subcutaneous implant type of the present invention is constructed as described below.
(1) A liquid medicine injection device of subcutaneous implant type comprising a device body composed of a ring-shaped upper member and a lower member closing a bottom surface of said upper member, a sealing member accommodated between said upper member and said lower member, closing an opening of said upper member and forming a liquid medicine inflow space inside said body, and a discharge port communicating with said liquid medicine inflow space, wherein said lower member has a bottom plate part; an annular wall part projected from said bottom plate part; a part, for pressing a peripheral portion of said sealing member, which is formed of an outer-edge side portion of an upper surface of said annular wall part; and an annular edge part which has an annular inclined surface formed at an inner-edge side portion of said upper surface of said annular wall part, upward projecting, and upward increasing in an inner diameter thereof and which can be inserted into said sealing member; said ring-shaped upper member has a body part accommodating said sealing member with a central portion of said sealing member being projected and an annular projected part, for pressing said peripheral portion of said sealing member, which is projected inward into said opening of said body part; said sealing member has a body part; a flange part, formed at a peripheral portion of said body part, which is to be pressed by a lower surface of said annular projected part of said upper member and by said upper surface of said annular wall part of said lower member; a part, formed below said flange part, which is to be inserted into said lower member; and a skirt-shaped projected part which is formed at a peripheral portion of a lower surface of said part to be inserted into said lower member and becomes thinner toward an end thereof; in said sealing member, an upper-surface side of said flange part thereof is pressed by said annular projected part of said upper member, and an outer-edge portion of a lower-surface side of said flange part is pressed by said part, for pressing said peripheral portion of said sealing member, which is formed of said upper surface of said annular wall part of said lower member, said annular edge part formed on said annular wall part of said lower member is penetrating into an inner-edge portion of said lower-surface side of said flange part; said sealing member deforms owing to a high compression applied thereto by said upper member and said lower member sandwiching said sealing member therebetween and owing to said penetration of said annular edge part of said lower member into said inner-edge portion of said lower-surface side of said flange part, so that there is a decrease in a height of said flange part, an increase in a height of said part to be inserted into said lower member, and a decrease of said skirt-shaped projected part, and an outer peripheral surface of a lower end of said part to be inserted into said lower member closely contacts an inner surface of said annular wall part of said lower member.
   Therefore, the liquid medicine injection device of subcutaneous implant type of the present invention has not a gap formed between the inner surface of the lower member accommodating the lower portion of the sealing member (septum) and the peripheral portion of the lower surface of the sealing member (septum) and which is capable of preventing the generation of thrombus and the like attributable to the penetration of blood into the gap.
   The embodiments of the present invention may be carried out as described below.
(2) A liquid medicine injection device of subcutaneous implant type according to the above (1), wherein said annular projected part of said upper member has an annular edge portion which has an annular inclined surface formed on an inner-edge side portion of said lower surface of said annular projected part, projecting downward, and downward increasing in an inner diameter thereof and which can be inserted into said flange part of said sealing member; and a portion, for pressing said peripheral portion of said sealing member, which is formed at an outer side of said annular edge portion.
(3) A liquid medicine injection device of subcutaneous implant type according to the above (2), wherein said sealing member has a part, to be inserted into said upper member, which is formed above said flange part;
   said sealing member deforms owing to a high compression applied thereto by said upper member and said lower member and owing to penetration of said annular edge part of said upper member into said sealing member, so that there is a decrease in a height of said flange part, an increase in a height of said part to be inserted into said upper member, and said part to be inserted into said upper member is projected above said opening of said upper member.
(4) A liquid medicine injection device of subcutaneous implant type according to any one of these above (1) through (3), wherein said lower surface of said part to be inserted into said lower member of said sealing member is formed as a rough surface.
(5) A liquid medicine injection device of subcutaneous implant type according to any one of these above (1) through (4), wherein said lower surface of said part to be inserted into said lower member of said sealing member is coated with an antithrombotic compound.
(6) A liquid medicine injection device of subcutaneous implant type according to any one of these above (1) through (5), wherein an inner surface of said lower member forming said liquid medicine inflow space is coated with an antithrombotic compound.
(7) A liquid medicine injection device of subcutaneous implant type according to any one of these above (1) through (6), wherein owing to said pressurization applied to said sealing member by said upper member and said lower member and owing to said penetration of said annular edge part of said lower member into said sealing member, said sealing member deforms and said skirt-shaped projected part thereof almost disappears.
(8) A liquid medicine injection device of subcutaneous implant type according to any one of these above (1) through (7), wherein said annular wall part of said lower member provides a discharge port mounting portion into which a proximal portion of said discharge port is inserted.
(9) A liquid medicine injection device of subcutaneous implant type according to any one of these above (1) through (8), comprising a contrast member disposed inside said device body.
(10) A liquid medicine injection device of subcutaneous implant type according to the above (9), wherein said flange part of said sealing member is pressed by said lower surface of said annular projected part of said upper member and by said upper surface of said annular wall part of said lower member, so that a height of said flange part decreases by 20 to 45%; and said contrast member has a clipped portion consisting of a mark which allows association of a contrast CT in which a contrast medium is administered to a patient.
(11) A liquid medicine injection device of subcutaneous implant type according to these above (9) or (10), wherein said contrast member is a ring-shaped member having a missing portion and is wide at a central portion of a rear side thereof.
(12) A liquid medicine injection device of subcutaneous implant type according to the above (10), wherein said clipped portion is left-right asymmetric.

## Claims

1. A liquid medicine injection device of subcutaneous implant type comprising a device body composed of a ring-shaped upper member and a lower member closing a bottom surface of said upper member, a sealing member accommodated between said upper member and said lower member, closing an opening of said upper member and forming a liquid medicine inflow space inside said body, and a discharge port communicating with said liquid medicine inflow space,
wherein said lower member has a bottom plate part; an annular wall part projected from said bottom plate part; a part, for pressing a peripheral portion of said sealing member, which is formed of an outer-edge side portion of an upper surface of said annular wall part; and an annular edge part which has an annular inclined surface formed at an inner-edge side portion of said upper surface of said annular wall part, upward projecting, and upward increasing in an inner diameter thereof and which can be inserted into said sealing member;
said ring-shaped upper member has a body part accommodating said sealing member with a central portion of said sealing member being projected and an annular projected part, for pressing said peripheral portion of said sealing member, which is projected inward into said opening of said body part;
said sealing member has a body part; a flange part, formed at a peripheral portion of said body part, which is to be pressed by a lower surface of said annular projected part of said upper member and by said upper surface of said annular wall part of said lower member; a part, formed below said flange part, which is to be inserted into said lower member; and a skirt-shaped projected part which is formed at a peripheral portion of a lower surface of said part to be inserted into said lower member and becomes thinner toward an end thereof;
in said sealing member, an upper-surface side of said flange part thereof is pressed by said annular projected part of said upper member, and an outer-edge portion of a lower-surface side of said flange part is pressed by said part, for pressing said peripheral portion of said sealing member, which is formed of said upper surface of said annular wall part of said lower member, said annular edge part formed on said annular wall part of said lower member is penetrating into an inner-edge portion of said lower-surface side of said flange part;
said sealing member deforms owing to a high compression applied thereto by said upper member and said lower member sandwiching said sealing member therebetween and owing to said penetration of said annular edge part of said lower member into said inner-edge portion of said lower-surface side of said flange part, so that there is a decrease in a height of said flange part, an increase in a height of said part to be inserted into said lower member, and a decrease of said skirt-shaped projected part, and an outer peripheral surface of a lower end of said part to be inserted into said lower member closely contacts an inner surface of said annular wall part of said lower member.

2. A liquid medicine injection device of subcutaneous implant type according to claim 1, wherein said annular projected part of said upper member has an annular edge portion which has an annular inclined surface formed on an inner-edge side portion of said lower surface of said annular projected part, projecting downward, and downward increasing in an inner diameter thereof and which can be inserted into said flange part of said sealing member; and a portion, for pressing said peripheral portion of said sealing member, which is formed at an outer side of said annular edge portion.

3. A liquid medicine injection device of subcutaneous implant type according to claim 2, wherein said sealing member has a part, to be inserted into said upper member, which is formed above said flange part;
said sealing member deforms owing to a high compression applied thereto by said upper member and said lower member and owing to penetration of said annular edge part of said upper member into said sealing member, so that there is a decrease in a height of said flange part, an increase in a height of said part to be inserted into said upper member, and said part to be inserted into said upper member is projected above said opening of said upper member.

4. A liquid medicine injection device of subcutaneous implant type according to any one of claims 1 through 3, wherein said lower surface of said part to be inserted into said lower member of said sealing member is formed as a rough surface.

5. A liquid medicine injection device of subcutaneous implant type according to any one of claims 1 through 4, wherein said lower surface of said part to be inserted into said lower member of said sealing member is coated with an antithrombotic compound.

6. A liquid medicine injection device of subcutaneous implant type according to any one of claims 1 through 5, wherein an inner surface of said lower member forming said liquid medicine inflow space is coated with an antithrombotic compound.

7. A liquid medicine injection device of subcutaneous implant type according to any one of claims 1 through 6, wherein owing to said pressurization applied to said sealing member by said upper member and said lower member and owing to said penetration of said annular edge part of said lower member into said sealing member, said sealing member deforms and said skirt-shaped projected part thereof almost disappears.

8. A liquid medicine injection device of subcutaneous implant type according to any one of claims 1 through 7, wherein said annular wall part of said lower member provides a discharge port mounting portion into which a proximal portion of said discharge port is inserted.

9. A liquid medicine injection device of subcutaneous implant type according to any one of claims 1 through 8, comprising a contrast member disposed inside said device body.

10. A liquid medicine injection device of subcutaneous implant type according to claim 9, wherein said flange part of said sealing member is pressed by said lower surface of said annular projected part of said upper member and by said upper surface of said annular wall part of said lower member, so that a height of said flange part decreases by 20 to 45%; and said contrast member has a clipped portion consisting of a mark which allows association of a contrast CT in which a contrast medium is administered to a patient.

11. A liquid medicine injection device of subcutaneous implant type according to claim 9 or 10, wherein said contrast member is a ring-shaped member having a missing portion and is wide at a central portion of a rear side thereof.

12. A liquid medicine injection device of subcutaneous implant type according to claim 10, wherein said clipped portion is left-right asymmetric.
